(19) Europäisches Patentamt / European Patent Office / Office européen des brevets

(11) EP 1 898 709 B1

# (12) EUROPEAN PATENT SPECIFICATION

(45) Date of publication and mention of the grant of the patent:
**20.01.2016 Bulletin 2016/03**

(21) Application number: **06763795.9**

(22) Date of filing: **20.06.2006**

(51) Int Cl.:
*A01N 59/26* (2006.01) *A01N 57/12* (2006.01)
*A01N 37/36* (2006.01) *A01N 37/38* (2006.01)
*A01N 37/18* (2006.01) *A61P 3/00* (2006.01)
*A01N 61/00* (2006.01) *A01N 47/04* (2006.01)
*A01N 37/46* (2006.01)

(86) International application number:
**PCT/EP2006/063349**

(87) International publication number:
**WO 2006/136551 (28.12.2006 Gazette 2006/52)**

## (54) FUNGICIDE COMPOSITION COMPRISING A PHOSPHOROUS ACID DERIVATIVE, A MANDELAMIDE TYPE COMPOUND AND A FURTHER FUNGICIDE COMPOUND

FUNGIZIDE ZUSAMMENSETZUNG ENTHALTEND EIN PHOSPORSÄURE DERIVAT, EINE VERBINDUNG DES TYPS MANDELAMID UND EINE WEITERE FUNGIZIDE VERBINDUNG

COMPOSITION FONGICIDE CONTENANT UN DÉRIVÉ DE L'ACID PHOSPHOREUX, UN COMPOSÉ DE TYPE MANDELAMIDE ET UN AUTRE COMPOSÉ FONGICIDE

(84) Designated Contracting States:
**AT BE BG CH CY CZ DE DK EE ES FI FR GB GR HU IE IS IT LI LT LU LV MC NL PL PT RO SE SI SK TR**

(30) Priority: **21.06.2005 EP 05356110**

(43) Date of publication of application:
**19.03.2008 Bulletin 2008/12**

(73) Proprietor: **Bayer Intellectual Property GmbH**
**40789 Monheim am Rhein (DE)**

(72) Inventors:
- **GOUOT, Jean-Marie**
  **F-69450 Saint Cyr Au Mont D'or (FR)**
- **LATORSE, Marie-Pascale**
  **F-69490 Saint Romain De Popey (FR)**

(74) Representative: **BIP Patents**
**c/o Bayer Intellectual Property GmbH**
**Alfred-Nobel-Straße 10**
**40789 Monheim am Rhein (DE)**

(56) References cited:
**EP-A2- 0 230 209** **WO-A-01/87822**
**WO-A-03/041728** **WO-A-2004/049804**
**WO-A-2005/041653** **WO-A2-2004/047540**

- **COUCH H B ET AL: "SYNERGISTIC AND ANTAGONISTIC INTERACTIONS OF FUNGICIDES AGAINST PYTHIUM APHANIDERMATUM ON PERENNIAL RYEGRASS" CROP PROTECTION, ELSEVIER SCIENCE, GB, vol. 10, no. 5, October 1991 (1991-10), pages 386-390, XP002050973 ISSN: 0261-2194**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 898 709 B1

Printed by Jouve, 75001 PARIS (FR)

## Description

The invention relates to active compound combinations, in particular within a fungicide composition, which comprise a phosphorous acid derivative (phosphonate or phosphite derivative), a compound of the mandelamide type like mandipropamid and a further fungicide compound. Such combinations are very highly suitable for controlling phytopathogenic fungi.
It is already known that phosphonic acid and some of its derivatives (phosphonates), such as fosetyl-Al, have fungicidal properties. The activity of these compounds is not always adequate at certain application rates notably.
Further it is likewise known that some mandelamide type compounds like mandipropamid possess fungicidal properties. The activity of this compound is good, but in certain cases leave something to be desired at low application rates.
The citations WO 01/87822 A1 and WO 03/041728 disclose fungicidal mandelamide compounds which may be combined with other fungicides such as phosphonic acids or fosetyl-Al.
Synergistic combinations of mandipropamid with dithianinon or carboxamides are known from WO 2004/0459804 A2 and WO 2005/041653 A2.
Binary synergistic combinations of acylalanine fungicides with phosphoric acids or phosphorous acid are known from EP 230 209 A2 and WO 2004/047540 A2.
Couch & Smith, Crop Prot. Vol. 10 (5), 386 - 390 (1991), disclose various combinations of fungicides which are synergistic or antagonistic against Pythium aphanidermatum on perennial ryegrass. A combination of fosetyl-Al and metalaxyl was shown to be synergistic.
Since, moreover, the environmental and economic requirements imposed on modern-day fungicides are continually increasing, with regard, for example, to the spectrum of action, toxicity, selectivity, application rate, formation of residues, and favourable preparation ability, and since, furthermore, there may be problems, for example, with resistances, a constant task is to develop new fungicide agents which in some areas at least have advantages over their known counterparts.
The invention provides active compound compositions which in some aspects at least achieve the stated objectives.
Accordingly, the present invention provides a fungicide composition comprising:

A) a phosphorous acid derivative selected from fosetyl-aluminium and phosphorous acid;
B) mandipropamid; and
C) a further fungicide compound selected from folpet and mefenoxam

wherein the weight ratios of the compounds A : B : C are from the range 1 : 0.01 : 0.01 to the range 1 : 0.1 : 2.
It has been found that the composition according to the invention exhibits very good fungicidal properties. The composition according to the invention possesses a broaden spectrum of activity compared to the existing or known fungicide compositions.
Surprisingly the fungicide activity of the composition according to the invention is substantially higher than the sum of the activities of the individual active compounds. In other words there is an unforeseeable, true synergistic effect and not merely an addition of activities.
The composition according to the invention can also provide an improved systemicity to the active compounds that are used. Indeed, even if some of the used fungicide compounds do not possess any or a satisfying systemicity, within the composition according to the invention these compounds can exhibit such a property.
In a similar manner, the composition according to the invention can allow an increased persistence of the fungicide efficacy of the active compounds that are employed.
Another advantage of the composition according to the invention relies in that an increased curativity is accessible.
Phosphonic acid is referred to in the literature, *inter alia,* as phosphorous acid (actually a tautomer of phosphonic acid, $P(OH)_3$), and its salts, correspondingly, as (secondary) phosphites.
Preferably, the composition according to the invention comprises fosetyl-Al or phosphorous acid as compound A. More preferably, fosetyl-Al is chosen as compound A in the composition according to the invention. The chemical name of fosetyl is ethyl hydrogen phosphonate.
The compound mandipropamidhas the following chemical name:

4-chloro-alpha-propynyloxy-N-[2-[3-methoxy-4-(2-propynyloxy)phenyl]ethyl]-benzeneacetamide, which corresponds to the following chemical structure:

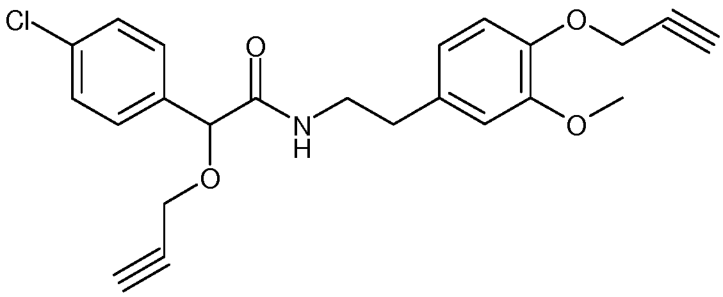

In the composition according to the invention, compound C is selected in the list consisting of

C1) a compound capable to inhibit the nucleic acid synthesis mefenoxam (formerly metalaxyl-M);

C13) a compound capable to have a multisite action - folpet.

The preferred composition according to the invention comprises

A) fosetyl-Al;
B) mandipropamid; and
C) a further fungicide compound selected in the list consisting of mefenoxam, and folpet.

The most preferred composition according to the invention combines fosetyl-Al, mandipropamid and folpet or mefenoxam.

A synergistic effect is particularly apparent when the active compounds are present in the composition according to the invention in defined weight ratios. The said weight ratios can be varied within a range. The weight ratios of the compounds A : B : C are situated in the range 1 : 0.01 : 0.01 and 1 : 0.1 : 2. Preferably these weight ratios are situated within the range 1 : 0.01 : 0.01 and 1 : 0.1 : 0.01.

The fungicide composition according to the invention may further comprise an agriculturally acceptable support, carrier or filler.

According to the invention, the term "support" denotes a natural or synthetic, organic or inorganic compound with which the active compound of formula (I) is combined or associated to make it easier to apply, notably to the parts of the plant. This support is thus generally inert and should be agriculturally acceptable. The support may be a solid or a liquid. Examples of suitable supports include clays, natural or synthetic silicates, silica, resins, waxes, solid fertilisers, water, alcohols, in particular butanol, organic solvents, mineral and plant oils and derivatives thereof. Mixtures of such supports may also be used.

The composition according to the invention may also comprise additional components. In particular, the composition may further comprise a surfactant. The surfactant can be an emulsifier, a dispersing agent or a wetting agent of ionic or non-ionic type or a mixture of such surfactants. Mention may be made, for example, of polyacrylic acid salts, lignosulphonic acid salts, phenolsulphonic or naphthalenesulphonic acid salts, polycondensates of ethylene oxide with fatty alcohols or with fatty acids or with fatty amines, substituted phenols (in particular alkylphenols or arylphenols), salts of sulphosuccinic acid esters, taurine derivatives (in particular alkyl taurates), phosphoric esters of polyoxyethylated alcohols or phenols, fatty acid esters of polyols, and derivatives of the present compounds containing sulphate, sulphonate and phosphate functions. The presence of at least one surfactant is generally essential when the active compound and / or the inert support are water-insoluble and when the vector agent for the application is water. Preferably, surfactant content may be comprised from 5% to 40% by weight of the composition.

Optionally, other additional components may also be included, e.g. protective colloids, adhesives, thickeners, thixotropic agents, penetration agents, stabilisers, sequestering agents. More generally, the active compounds can be combined with any solid or liquid additive, which complies with the usual formulation techniques.

In general, the composition according to the invention may contain from 0.05 to 99% by weight of active compounds, preferably from 10 to 70% by weight.

Compositions according to the invention can be used in various forms such as aerosol dispenser, capsule suspension, cold fogging concentrate, dustable powder, emulsifiable concentrate, emulsion oil in water, emulsion water in oil, encapsulated granule, fine granule, flowable concentrate for seed treatment, gas (under pressure),gas generating product, granule, hot fogging concentrate, macrogranule, microgranule, oil dispersible powder, oil miscible flowable concentrate, oil miscible liquid, paste, plant rodlet, powder for dry seed treatment, seed coated with a pesticide, soluble concentrate, soluble powder, solution for seed treatment, suspension concentrate (flowable concentrate), ultra low

volume (ULV) liquid, ultra low volume (ULV) suspension, water dispersible granules or tablets, water dispersible powder for slurry treatment, water soluble granules or tablets, water soluble powder for seed treatment and wettable powder.

These compositions include not only compositions which are ready to be applied to the plant or seed to be treated by means of a suitable device, such as a spraying or dusting device, but also concentrated commercial compositions which must be diluted before application to the crop.

The active compounds within the composition according to the invention have potent microbicide activity and can be employed for controlling undesired micro-organisms, such as fungi or bacteria, in crop protection or in the protection of materials.

Within the composition according to the invention, fungicide compounds can be employed in crop protection for example for controlling Plasmodiophoromycetes, Oomycetes, Chytridiomycetes, Zygomycetes, Ascomycetes, Basidiomycetes and Deuteromycetes.

Within the composition according to the invention, bactericide compounds can be employed in crop protection for example for controlling Pseudomonadaceae, Rhizobiaceae, Enterobacteriaceae, Corynebacteriaceae and Streptomycetaceae.

The fungicide composition according to the invention can be used to curatively or preventively control the phytopathogenic fungi of plants or crops. Thus, according to a further aspect of the invention, there is provided a method for curatively or preventively controlling the phytopathogenic fungi of plants or crops comprising the use of a fungicide composition according to the invention by application to the seed, the plant or to the fruit of the plant or to the soil in which the plant is growing or in which it is desired to grow.

The method of treatment according to the invention may also be useful to treat propagation material such as tubers or rhizomes, but also seeds, seedlings or seedlings pricking out and plants or plants pricking out. This method of treatment can also be useful to treat roots. The method of treatment according to the invention can also be useful to treat the over-ground parts of the plant such as trunks, stems or stalks, leaves, flowers and fruit of the concerned plant. The method of treatment according to the invention may also consist in the application or the use of compounds A, B and C as combined products, as alternate products or as sequence products.

Among the plants that can be protected by the method according to the invention, mention may be made of cotton; flax; vine; fruit or vegetable crops such as *Rosaceae sp.* (for instance pip fruit such as apples and pears, but also stone fruit such as apricots, almonds and peaches), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (for instance banana trees and plantins), *Rubiaceae sp., Theaceae sp., Sterculiceae sp., Rutaceae sp.* (for instance lemons, oranges and grapefruit); *Solanaceae sp.* (for instance tomatoes), *Liliaceae sp., Asteraceae sp.* (for instance lettuces), *Umbelliferae sp., Cruciferae sp., Chenopodiaceae sp., Cucurbitaceae sp., Papilionaceae sp.* (for instance peas), *Rosaceae sp.* (for instance strawberries); major crops such as *Graminae sp.* (for instance maize, lawn or cereals such as wheat, rice, barley and triticale), *Asteraceae sp.* (for instance sunflower), *Cruciferae sp.* (for instance colza), *Fabacae sp.* (for instance peanuts), *Papilionaceae sp.* (for instance soybean), *Solanaceae sp.* (for instance potatoes), *Chenopodiaceae sp.* (for instance beetroots); horticultural and forest crops; as well as genetically modified homologues of these crops.

Among the diseases of plants or crops that can be controlled by the method according to the invention, mention may be made of :

Powdery mildew diseases such as :

- Blumeria diseases, caused for example by *Blumeria graminis*;
- Podosphaera diseases, caused for example by *Podosphaera leucotricha;*
- Sphaerotheca diseases, caused for example by *Sphaerotheca fuliginea;*
- Uncinula diseases, caused for example by *Uncinula necator*;

Rust diseases such as :

- Gymnosporangium diseases, caused for example by *Gymnosporangium sabinae;*
- Hemileia diseases, caused for example by *Hemileia vastatrix;*
- Phakopsora diseases, caused for example by *Phakopsora pachyrhizi* or *Phakopsora meibomiae;*
- Puccinia diseases, caused for example by *Puccinia recondita;*
- Uromyces diseases, caused for example by *Uromyces appendiculatus;*

Oomycete diseases such as :

- Bremia diseases, caused for example by *Bremia lactucae;*
- Peronospora diseases, caused for example by *Peronospora pisi* or *P. brassicae;*

Phytophthora diseases, caused for example by *Phytophthora infestans*;
Plasmopara diseases, caused for example by *Plasmopara viticola*;
Pseudoperonospora diseases, caused for example by *Pseudoperonospora humuli or Pseudoperonospora cubensis*;
Pythium diseases, caused for example by *Pythium ultimum*;

Leafspot, leaf blotch and leaf blight diseases such as :

Alternaria diseases, caused for example by *Alternaria solani*;
Cercospora diseases, caused for example by *Cercospora beticola*;
Cladiosporum diseases, caused for example by *Cladiosporium cucumerinum*;
Cochliobolus diseases, caused for example by *Cochliobolus sativus;*
Colletotrichum diseases, caused for example by *Colletotrichum lindemuthanium;*
Cycloconium diseases, caused for example by *Cycloconium oleaginum*;
Diaporthe diseases, caused for example by *Diaporthe citri*;
Elsinoe diseases, caused for example by *Elsinoe fawcettii*;
Gloeosporium diseases, caused for example by *Gloeosporium laeticolor*;
Glomerella diseases, caused for example by *Glomerella cingulata*;
Guignardia diseases, caused for example by *Guignardia bidwelli*;
Leptosphaeria diseases, caused for example by *Leptosphaeria maculans*; *Leptosphaeria nodorum;*
Magnaporthe diseases, caused for example by *Magnaporthe grisea*;
Mycosphaerella diseases, caused for example by *Mycosphaerella graminicola*; *Mycosphaerella arachidicola; Mycosphaerella fijiensis;*
Phaeosphaeria diseases, caused for example by *Phaeosphaeria nodorum*;
Pyrenophora diseases, caused for example by *Pyrenophora teres*;
Ramularia diseases, caused for example by *Ramularia collo-cygni*;
Rhynchosporium diseases, caused for example by *Rhynchosporium secalis*;
Septoria diseases, caused for example by *Septoria apii* or *Septoria lycopercisi*;
Typhula diseases, caused for example by *Typhula incarnata*;
Venturia diseases, caused for example by *Venturia inaequalis*;

Root and stem diseases such as :

Corticium diseases, caused for example by *Corticium graminearum*;
Fusarium diseases, caused for example by *Fusarium oxysporum*;
Gaeumannomyces diseases, caused for example by *Gaeumannomyces graminis*;
Rhizoctonia diseases, caused for example by *Rhizoctonia solani*;
Tapesia diseases, caused for example by *Tapesia acuformis*;
Thielaviopsis diseases, caused for example by *Thielaviopsis basicola*;

Ear and panicle diseases such as :

Alternaria diseases, caused for example by *Alternaria spp.*;
Aspergillus diseases, caused for example by *Aspergillus flavus*;
Cladosporium diseases, caused for example by *Cladosporium spp.*;
Claviceps diseases, caused for example by *Claviceps purpurea*;
Fusarium diseases, caused for example by *Fusarium culmorum*;
Gibberella diseases, caused for example by *Gibberella zeae*;
Monographella diseases, caused for example by *Monographella nivalis*;

Smut and bunt diseases such as :

Sphacelotheca diseases, caused for example by *Sphacelotheca reiliana*;
Tilletia diseases, caused for example by *Tilletia caries;*
Urocystis diseases, caused for example by *Urocystis occulta*;
Ustilago diseases, caused for example by *Ustilago nuda*;

Fruit rot and mould diseases such as :

Aspergillus diseases, caused for example by *Aspergillus flavus*;
Botrytis diseases, caused for example by *Botrytis cinerea*;
Penicillium diseases, caused for example by *Penicillium expansum*;
Sclerotinia diseases, caused for example by *Sclerotinia sclerotiorum*;
Verticilium diseases, caused for example by *Verticilium alboatrum*;

Seed and soilborne decay, mould, wilt, rot and damping-off diseases :

Fusarium diseases, caused for example by *Fusarium culmorum*;
Phytophthora diseases, caused for example by *Phytophthora cactorum*;
Pythium diseases, caused for example by *Pythium ultimum*;
Rhizoctonia diseases, caused for example by *Rhizoctonia solani*;
Sclerotium diseases, caused for example by *Sclerotium rolfsii*;
Microdochium diseases, caused for example by *Microdochium nivale*;

Canker, broom and dieback diseases such as :

Nectria diseases, caused for example by *Nectria galligena*;

Blight diseases such as :

Monilinia diseases, caused for example by *Monilinia laxa*;

Leaf blister or leaf curl diseases such as :

Taphrina diseases, caused for example by *Taphrina deformans*;

Decline diseases of wooden plants such as :

Esca diseases, caused for example by *Phaemoniella clamydospora*;
Eutypa dyeback, caused for example by *Eutypa lata*;
Dutch elm disease, caused for example by *Ceratocystis ulmi*;

Diseases of flowers and Seeds such as :

Botrytis diseases, caused for example by *Botrytis cinerea*;

Diseases of tubers such as :

Rhizoctonia diseases, caused for example by *Rhizoctonia solani*.

The fungicide composition according to the invention may also be used against fungal diseases liable to grow on or inside timber. The term "timber" means all types of species of wood, and all types of working of this wood intended for construction, for example solid wood, high-density wood, laminated wood, and plywood. The method for treating timber according to the invention mainly consists in contacting one or more compounds according to the invention, or a composition according to the invention; this includes for example direct application, spraying, dipping, injection or any other suitable means.

The dose of active compound usually applied in the method of treatment according to the invention is generally and advantageously from 0.1 to 10,000 g / ha, preferably from 10 to 1000 g / ha. For seed dressing, the active-compound combination application rates are generally from 0.001 to 50 g per kilogram of seed, preferably from 0.01 to 10 g per kilogram of seed. For the treatment of the soil, the active-compound combination application rates are generally from 0.1 to 10,000 g / ha, preferably from 1 to 5,000 g / ha.

In the method according to the invention, compounds A, B and C according to the invention are preferably used in the following amounts:

A) the phosphorous acid derivative fosetyl-Al or phosphorous acid, in an amount from 500 to 1,500 g/ha;
B) mandipropamid in an amount from 20 to 150 g/ha, preferably from 50 to 75 g/ha;
C) the further fungicide compound in an amount from 25 to 3,000 g/ha.

Particular examples of combinations according to the method of treatment according to the invention comprise the use of

A) fosetyl-Al used in an amount from 500 to 2,000 g/ha;
B) mandipropamid used in an amount from 20 to 150 g/ha, preferably from 50 to 75 g/ha; and
C) a further fungicide compound selected in the list consisting of

- mefenoxam used in an amount from 50 to 120 g/ha, preferably from 50 to 75 g/ha, folpet used in an amount from 500 to 1,000 g/ha, preferably from 500 to 750 g/ha.

It is clearly understood that the doses indicated herein are given as illustrative examples of the method according to the invention. A person skilled in the art will know how to adapt the application doses, notably according to the nature of the plant or crop to be treated.

The fungicide composition according to the invention may also be used in the treatment of genetically modified organisms with the compounds according to the invention or the agrochemical compositions according to the invention. Genetically modified plants are plants into genome of which a heterologous gene encoding a protein of interest has been stably integrated. The expression "heterologous gene encoding a protein of interest" essentially means genes which give the transformed plant new agronomic properties, or genes for improving the agronomic quality of the modified plant.

The following examples are given purely by way of illustration of the invention without any limitation. They are intended to give an illustration of the efficacy of the 3-way or ternary composition according to the invention on plant diseases.

<u>Example 1</u>

Synergistic effect of the mixture of fosetyl-Al (compound A) combined with mandipropamid (compound B) and folpet (compound C) in preventive 72h application on vine downy mildew has been demonstrated.

Materials and Methods

Products :

Table 1 : compound specifications

| Product | a.i. | a.i. concentration | Formulation |
|---|---|---|---|
| A: Aliette | fosetyl-Al | 80% | WG |
| B: formulated mandipropamid | mandipropamid | 10% | SC |
| C : Acryptane 500 | folpet | 50% | SC |

Methods :

Vine plants (variety Cabernet Sauvignon) were grown in a sandy soil in plastic pots with a single plant per pot. At the age of 2 months (6 to 7 leaves developed), these plants were sprayed with the three fungicide active compounds either alone or in mixture. Fungicide active compounds applied in mixture were also applied individually at the same doses as those used in the combinations.

The fungicide active compounds, either used alone or in mixture, were applied at a volume rate of 250 l water per ha.

The following ratio was tested : fosetyl-Al (A) / mandipropamid (B) / folpet (C) respectively: 1,500 g a.i. /ha +75 g a.i. /ha + 750 g a.i. /ha (ratio 20 / 1 /10).

The mixture of fosetyl-Al with mandipropamid and folpet was prepared either with fosetyl-Al in the form of water-dispersible granules (WG) at 800 g/Kg and mandipropamid in the form of concentrated suspension (SC) at 100 g/L and folpet in the form of concentrated suspension (SC) at 500 g/L.

The dose range tested for fosetyl-Al was: 2,500 g a.i. /ha, 1,500 g a.i. /ha, 1,000 g a.i. /ha, 750 g a.i. /ha, 500 g a.i. /ha, 250 g a.i. /ha, 150 g a.i. /ha, 15 g a.i. /ha, 3 g a.i. /ha, 1.5 g a.i. /ha, 0.15 g a.i. /ha and 0.015 g a.i. /ha.

The dose range for mandipropamid + folpet was: 75 + 750 g a.i. /ha, 1 + 10 g a.i. /ha, 0.1 + 1 g a.i. /ha, 0.02 + 0.2, 0.01 + 0.1 g a.i. /ha, 0.001 + 0.01 g a.i. /ha and 0.0001 + 0.001 g a.i. /ha. For the ternary mixture fosetyl-Al + mandipropamid + folpet, the dose range studied was: 1,500 + 75 + 750 g a.i. /ha, 20 + 1+ 10 g a.i. /ha, 2 + 0.1 + 1 g a.i. /ha, 0.4 + 0.02 + 0.2 g a.i. /ha, 0.2 + 0.01 + 0.1 g a.i. /ha, 0.02 + 0.001 + 0.01 g a.i. /ha and 0.002 + 0.0001 + 0.001 g a.i. /ha.

The UTC plants were only treated with water without any active compound.

A three days interval separated treatment and inoculation. Three days after the treatment, each plant was inoculated by spraying an aqueous suspension of sporangia of *Plasmopara viticola* obtained from infected leaves and sensitive to all the tested compounds. The concentration of sporangia was about 100,000 units per ml.

After contamination the plants were incubated for 7 days at 22°C in a saturated atmosphere. Seven days after contamination, symptoms were evaluated in terms of the extent of the lower surface of the leaves infected, in comparison with untreated (UTC) but contamined plants.

The efficacy of the treatment was calculated using the Abbott formula :

$$\text{efficacy} = (\text{ untreated - treated / untreated })\text{x } 100$$

The concentrations of the fungicide active substances alone or in mixture giving % efficacy for each component were determined based on the sigmoid curve dose / response model with their confidence intervals. The results corresponding to ED90, ED70 or ED50 (effective doses providing 90%, 70% or 50% of disease control) for the ratio studied have been calculated on the basis of 4 repeats per factor. The analysis of the results was made according to the TAMMES model. The ED90, ED70 or ED50 values observed for each compound alone or in mixture were then inserted in a graphic representation as described by TAMMES (Isoboles, a graphic representation of synergism in pesticides, Neth.J. Plant Path. 70 (1964): 73-80).

Each of the components is expressed in a coordinate. The line which connects the points is called an isobole. With an isobole, the effect of different proportions of the components can be calculated. The observed value for the mixture corresponding to one ratio A / B is compared to the theoretical value calculated via the isobole of additive effect. In that case, the total effect observed is equal to the sum of the effects of the components taken independently.

A synergistic effect or a cooperative action of two or more compounds in mixture means that the total effect is greater or more prolonged than the sum of effects of the products taken independently.

Results:

For the ternary mixture fosetyl-Al / mandipropamid / folpet, the results obtained are presented in the form of points values, corresponding to 90%, 70% or 50% control of the pathogen and placed in a TAMMES isobole diagram which comprises on the x-axis the doses of fosetyl-Al in g a.i. / ha and on the y-axis the doses of the mixture mandipropamid with folpet expressed in g a.i. / ha. These results are presented in a table with the quantity of each compound in the ternary mixture at the studied ratio (A / B / C) practically observed to control 90%, 70% or 50% of the disease compared to the theoretical quantity of each, expected to supply the same control in the case of no synergy between the 3 active substances.

For the ratio 20 / 1 / 10 corresponding to fosetyl-Al with mandipropamid and folpet, the preventive test done on grapevine downy mildew shows synergistic effect of the compositions comprising fosetyl-Al with mandipropamid and folpet. It has been demonstrated that less amount of both compounds are necessary to effectively control 90%, 70% and 50% of the disease compared to the theoretical doses of each compounds expected in the mixture with just additional effect.

In table 2, EC50, EC70, EC90 calculated according to efficacy observed for this ratio illustrate these results.

Table 2 : Treatment of vine cuttings with a composition according to the invention - Synergism of a mixture of fosetyl-Al with mandipropamid and folpet according to TAMMES method for 90%, 70% and 50% of disease control.

| | fosetyl-Al + mandipropamid + folpet g a.i. / ha | mandipropamid + folpet g a.i. / ha | fosetyl-Al g a.i. / ha |
|---|---|---|---|
| Ratio | 20 / 1 / 10 | 1 / 10 | |
| Practical dose for 90% efficacy | 14.6 / 0.73 / 7.3 (= 22.63) | 1.27 / 12.7 (= 13.97) | 655 |
| Theoretical dose for 90% efficacy | 25.4/1.27/12.7 (= 39.37) | / | / |

(continued)

| | fosetyl-Al + mandipropamid + folpet g a.i. / ha | mandipropamid + folpet g a.i. / ha | fosetyl-Al g a.i. / ha |
|---|---|---|---|
| Synergy for 90% efficacy (1-practical dose / theoretical dose) | 40% | | |
| Practical dose for 70% efficacy | 2.4 / 0.12 / 1.2 (= 3.72) | 0.23 / 2.3 (= 2.53) | 125 |
| Theoretical dose for 70% efficacy | 4.7/0.23/2.3 (= 7.23) | / | / |
| synergy for 70% efficacy (1-practical dose / theoretical dose) | 50% | | |
| Practical dose for 50% efficacy | 0.72 / 0.036 / 0.36 (=1.116) | 0.08 / 0.76 (= 0.84) | 44 |
| Theoretical dose for 50% efficacy | 1.6/0.08/0.76 (= 2.44) | / | / |
| Synergy for 50% efficacy (1-practical dose / theoretical dose) | 55% | | |

Conclusion

In these greenhouse studies, synergy for a mixture of fosetyl-Al wit mandipropamid and folpet was demonstrated in 72h preventive situation for the ratio 20 / 1 / 10 according to TAMMES model.

<u>Example 2</u>

Synergistic effect of the mixture of fosetyl-Al (compound A) combined with mandipropamid (compound B) and mefenoxam (compound C) in preventive 72h application on vine downy mildew has been demonstrated.

Materials and Methods

Products :

Table 1 : compound specifications

| Product | a.i. | a.i. concentration | Formulation |
|---|---|---|---|
| A: Aliette | fosetyl-Al | 80% | WG |
| B: formulated mandipropamid | mandipropamid | 10% | SC |
| C: Ridomyl gold | mefenoxam | 480 g /L | EC |

Methods :

Vine plants (variety Cabernet Sauvignon) were grown in a sandy soil in plastic pots with a single plant per pot. At the age of 2 months (6 to 7 leaves developed), these plants were sprayed with the three fungicide active compounds either alone or in mixture. Fungicide active compounds applied in mixture were also applied individually at the same doses as those used in the combinations.

The fungicide active compounds, either used alone or in mixture, were applied at a volume rate of 250 l water per ha.

The following ratio was tested: fosetyl-Al (A) / mandipropamid (B) / mefenoxam (C) respectively: 1,500 g a.i. / ha + 75 g a.i. / ha + 75 g a.i. / ha (ratio 20 / 1 / 1).

The dose range tested for fosetyl-Al was : 2,500 g a.i. / ha,1,500g a.i. / ha, 750 g a.i. / ha, 500 g a.i. / ha, 250 g a.i. / ha, 150 g a.i. / ha, 50 g a.i. / ha, 30 g a.i. / ha, 10 g a.i. / ha, 5 g a.i. / ha and 0.5 g a.i. / ha.

The dose range for mandipropamid + mefenoxam was: 75 + 75 g a.i. / ha, 0.75 + 0.75 g a.i. / ha, 0.25 + 0.25 g a.i. / ha, 0.15 + 0.15 g a.i. / ha , 0.05 + 0.05 g a.i. / ha, 0.025 + 0.025 g a.i. / ha and 0.0025 + 0.0025 g a.i. / ha.

For the ternary mixture fosetyl-Al + mandipropamid + mefenoxam, the dose range studied was: 1,500 + 75 + 75 g a.i. / ha, 150 + 0.75 + 0.75 g a.i. / ha, 50 + 0.25 + 0.25 g a.i. / ha, 30 + 0.15 + 0.15 g a.i. / ha, 10 + 0.05 + 0.05 g a.i. / ha, 5 + 0.025 + 0.025 g a.i. / ha and 0.5 + 0.0025 + 0.0025 g a.i. / ha.

The UTC plants were only treated with water without any active compound.

For managing vapour effect of mefenoxam, all the different factors were separated and isolated after treatment all along the test in independent greenhouse boxes.

A three days interval separated treatment and inoculation. Three days after the treatment, each plant was inoculated by spraying an aqueous suspension of sporangia of *Plasmopara viticola* obtained from infected leaves and sensitive to all the tested compounds. The concentration of sporangia was about 100,000 units per ml.

After contamination the plants were incubated for 7 days at 22°C in a saturated atmosphere. Seven days after contamination, symptoms were evaluated in terms of the extent of the lower surface of the leaves infected, in comparison with untreated (UTC) but contamined plants.

The efficacy of the treatment was calculated using the Abbott formula :

$$\text{efficacy} = (\text{ untreated - treated / untreated })\text{x } 100$$

The concentrations of the fungicide active substances alone or in mixture giving % efficacy for each component were determined based on the sigmoid curve dose / response model with their confidence intervals. The results corresponding to ED90, ED70 or ED50 (effective doses providing 90%, 70% or 50% of disease control) for the ratio studied have been calculated on the basis of 4 repeats per factor. The analysis of the results was made according to the TAMMES model. The ED90, ED70 or ED50 values observed for each compound alone or in mixture were then inserted in a graphic representation as described by TAMMES (Isoboles, a graphic representation of synergism in pesticides, Neth.J. Plant Path. 70 (1964): 73-80).

Each of the components is expressed in a coordinate. The line which connects the points is called an isobole. With an isobole, the effect of different proportions of the components can be calculated. The observed value for the mixture corresponding to one ratio A / B is compared to the theoretical value calculated via the isobole of additive effect. In that case, the total effect observed is equal to the sum of the effects of the components taken independently.

A synergistic effect or a cooperative action of two or more compounds in mixture means that the total effect is greater or more prolonged than the sum of effects of the products taken independently.

Results:

For the ternary mixture fosetyl-Al / mandipropamid / mefenoxam, the results obtained are presented in the form of points values, corresponding to 90%, 70% or 50% control of the pathogen and placed in a TAMMES isobole diagram which comprises on the x-axis the doses of fosetyl-Al in g a.i. / ha and on the y-axis the doses of the mixture mandipropamid with mefenoxam expressed in g a.i. / ha. These results are presented in a table with the quantity of each compound in the ternary mixture at the studied ratio (A / B / C) practically observed to control 90%, 70% or 50% of the disease compared to the theoretical quantity of each, expected to supply the same control in the case of no synergy between the 3 active substances.

For the ratio 20 / 1 / 1 corresponding to fosetyl-Al with mandipropamid and mefenoxam, the preventive test done on grapevine downy mildew shows synergistic effect of the compositions comprising fosetyl-Al with mandipropamid and mefenoxam. It has been demonstrated that less amount of both compounds are necessary to effectively control 90%, 70% and 50% of the disease compared to the theoretical doses of each compounds expected in the mixture with just additional effect.

In table 2, EC50, EC70, EC90 calculated according to efficacy observed for this ratio illustrate these results.

Table 2 : Treatment of vine cuttings with a composition according to the invention - Synergism of a mixture of fosetyl-Al with mandipropamid and mefenoxam according to TAMMES method for 90%, 70% and 50% of disease control.

| | fosetyl-Al + mandipropamid + mefenoxam g a.i. / ha | mandipropamid + mefenoxam g a.i. / ha | fosetyl-Al g a.i. / ha |
|---|---|---|---|
| Ratio | 20 / 1 / 1 | 1 / 1 | |
| Practical dose for 90% efficacy | 0.8 / 0.042 / 0.042 (= 0.884) | 0.055 / 0.055 (= 0.11) | 1,500 |
| Theoretical dose for 90% efficacy | 1.1 / 0.055 / 0.055 (= 1.21) | / | / |
| Synergy for 90% efficacy (1-practical dose / theoretical dose) | 30% | | |
| Practical dose for 70% efficacy | 0.45 / 0.022 / 0.022 (= 0.494) | 0.026 / 0.026 (= 0.053) | 400 |
| Theoretical dose for 70% efficacy | 0.53 / 0.026 / 0.026 (= 0.582) | / | / |
| synergy for 70% efficacy (1-practical dose / theoretical dose) | 20% | | |
| Practical dose for 50% efficacy | 0.3 / 0.015 / 0.015 (= 0.33) | 0.017 / 0.017 (= 0.034) | 250 |
| Theoretical dose for 50% efficacy | 0.34 / 0.017 / 0.017 (= 0.374) | / | / |
| Synergy for 50% efficacy (1-practical dose / theoretical dose) | 10% | | |

Conclusion

In these greenhouse studies, synergy for a mixture of fosetyl-Al wit mandipropamid and mefenoxam was demonstrated in 72h preventive situation for the ratio 20 / 1 / 1 according to TAMMES model.

Example 3

Synergistic effect of the mixture phosphorous acid ($H_3PO_3$) (compound A) combined with mandipropamid (compound B) and folpet (compound C) in preventive 72h application on vine downy mildew has been demonstrated.

Materials and Methods

Products :

Table 1 : compound specifications

| Product | a.i. | a.i. concentration | Formulation |
|---|---|---|---|
| A: Prograp pK2 | Phosphorous oxide + potassium oxide | 320 g/L +120 g/ L | SL |
| B: formulated mandipropamid | mandipropamid | 10% | SC |
| C : Acryptane 500 | folpet | 50% | SC |

Methods :

Vine plants (variety Cabernet Sauvignon) were grown in a sandy soil in plastic pots with a single plant per pot. At the age of 2 months (6 to 7 leaves developed), these plants were sprayed with the three fungicide active compounds

either alone or in mixture. Fungicide active compounds applied in mixture were also applied individually at the same doses as those used in the combinations.

The fungicide active compounds, either used alone or in mixture, were applied at a volume rate of 250 l water per ha.

Vine plants (variety Cabernet Sauvignon) were grown in a sandy soil in plastic pots with a single plant per pot. At the age of 2 months (6 to 7 leaves developed), these plants were sprayed with the three fungicide active compounds either alone or in mixture. Fungicide active compounds applied in mixture were also applied individually at the same doses as those used in the combinations.

The fungicide active compounds, either used alone or in mixture, were applied at a volume rate of 250 l water per ha.

The following ratio was tested: phosphorous acid (A) / mandipropamid (B) / folpet (C) respectively: 1,500 g a.i. /ha + 75 g a.i. /ha + 750 g a.i. /ha (ratio 20 / 1 / 10).

The dose range tested for phosphorous acid equivalent (329 g/kg) was: 3,000 g a.i. /ha, 1,500 g a.i. /ha, 1,000 g a.i. /ha, 750 g a.i. /ha, 500 g a.i. /ha, 250 g a.i. /ha, 80 g a.i. /ha, 40 g a.i. /ha, 20 g a.i. /ha, 10 g a.i. /ha, 2 g a.i. /ha and 0.2 g a.i. /ha.

The dose range for mandipropamid + folpet was : 75 + 750 g a.i. /ha, 4 + 40 g a.i. /ha, 2 + 20 g a.i. /ha, 1 + 10 g a.i. /ha , 0.5 + 5 g a.i. /ha, 0.1 + 1 g a.i. /ha and 0.01 + 0.1g a.i. /ha.

For the ternary mixture phosphorous acid equivalent + mandipropamid + folpet, the dose range studied was: 1,500 + 75 + 750 g a.i. /ha, 80 + 4 + 40 g a.i. /ha, 40 + 2 + 20 g a.i. /ha, 20 + 1 + 10 g a.i. /ha, 10 + 0.5 + 5 g a.i. /ha, 2 + 0.1 + 1 g a.i. /ha and 0.2 + 0.01 + 0.1 g a.i. /ha.

The UTC plants were only treated with water without any active compound.

Four days before treatment, phosphorous acid was applied at 1,500 g a.i. /ha on plants planned to be treated later with phosphorous acid based product alone or in mixture.

A three days interval separated treatment and inoculation. Three days after the treatment, each plant was inoculated by spraying an aqueous suspension of sporangia of *Plasmopara viticola* obtained from infected leaves and sensitive to all the tested compounds. The concentration of sporangia was about 100,000 units per ml.

After contamination the plants were incubated for 7 days at 22°C in a saturated atmosphere. Seven days after contamination, symptoms were evaluated in terms of the extent of the lower surface of the leaves infected, in comparison with untreated (UTC) but contamined plants.

The efficacy of the treatment was calculated using the Abbott formula :

$$\text{efficacy} = (\text{untreated} - \text{treated} / \text{untreated}) \times 100$$

The concentrations of the fungicide active substances alone or in mixture giving % efficacy for each component were determined based on the sigmoid curve dose / response model with their confidence intervals. The analysis of the results was made according to the TAMMES model (Isoboles, a graphic representation of synergism in pesticides, Neth.J. Plant Path. 70 (1964): 73-80).

Results:

The calculated results corresponding to ED90 or ED70 (effective doses providing 90% or 70% of disease control) for the ratio that have been evaluated are presented in tables 2 and 3. These results have been calculated on the basis of 4 repeats per factor according to the TAMMES method.

The results obtained are presented in the form of points values, corresponding to 90% or 70% control of the pathogen and placed in a TAMMES isobole diagram which comprises on the x-axis the doses of phosphorous acid equivalent expressed in g a.i./ha and on the y-axis the doses of mandipropamid and folpet.

Table 2 : EC70 and EC90 calculated according to efficacy observed.

| | ED70 g a.i. / ha | ED90 g a.i. / ha |
|---|---|---|
| A : phosphorous acid | 6.2 | 145 |
| B + C : mandipropamid + folpet | 1.85 | 5 |
| A + B + C: phosphorous acid + mandipropamid + folpet | 0.07 | 0.4 |

Table 3 : Treatment of vine cuttings with a composition according to the invention - Synergism of a mixture of phosphorous acid with mandipropamid and folpet according to TAMMES method for 90% and 70% of disease control.

| | phosphorous acid + mandipropamid + folpet g a.i. / ha | mandipropamid + folpet g a.i. / ha | phosphorous acid g a.i. / ha |
|---|---|---|---|
| Ratio | 20 / 1 / 10 | 1 / 10 | / |
| Practical dose for 90% efficacy | 0.72/0.036/0.36 (= 1.116) | 0.43 / 4.3 (= 4.73) | 145 |
| Theoretical dose for 90% efficacy | 8.6 / 0.43 / 4.3 (= 13.33) | 0.43 / 4.3 | / |
| Synergy for 90% efficacy (1- practical dose / theoretical dose) | 90% | | |
| Practical dose for 70% efficacy | 0.1 / 0.006 / 0.6 (= 0.706) | 0.15 / 1.5 (= 1.65) | 6.2 |
| Theoretical dose for 70% efficacy | 3/0.15/1.5 (= 1.65) | / | / |
| Synergy for 70% efficacy (1- practical dose / theoretical dose) | > 90% | | |

Conclusion

In these greenhouse studies, synergy for a mixture of phosphorous acid wit mandipropamid and folpet was demonstrated in 72h preventive situation for the ratio 20 / 1 / 10 according to TAMMES model.

Example 4

Synergistic effect of the mixture phosphorous acid (compound A) combined with mandipropamid (compound B) and mefenoxam (compound C) in preventive 72h application on vine downy mildew has been demonstrated.

Materials and Methods

Products :

Table 1 : compound specifications

| Product | a.i. | a.i. concentration | Formulation |
|---|---|---|---|
| A: Prograp pK2 | Phosphorous oxide + potassium oxide | 320 g/L +120 g/ L | SL |
| B: formulated mandipropamid | mandipropamid | 10% | SC |
| C : Ridomyl gold | mefenoxam | 480 g/L | EC |

Methods :

Vine plants (variety Cabernet Sauvignon) were grown in a sandy soil in plastic pots with a single plant per pot. At the age of 2 months (6 to 7 leaves developed), these plants were sprayed with the three fungicide active compounds either alone or in mixture. Fungicide active compounds applied in mixture were also applied individually at the same doses as those used in the combinations.

The fungicide active compounds, either used alone or in mixture, were applied at a volume rate of 250 l water

per ha.

The following ratio was tested: phosphorous acid (A) / mandipropamid (B) / mefenoxam (C) respectively: 1,500 g a.i. /ha + 75 g a.i. /ha + 75 g a.i. /ha (ratio 20 / 1 / 1).

The dose range tested for phosphorous acid equivalent (329 g/kg) was: 2,500 g a.i. /ha, 1,500g a.i. /ha, 750 g a.i. /ha, 500 g a.i. /ha, 250 g a.i. /ha, 150 g a.i. /ha, 50 g a.i. /ha, 30 g a.i. /ha, 10 g a.i. /ha, 5 g a.i. /ha, and 0.5 g a.i. /ha.

The dose range for mandipropamid + mefenoxam was: 75 + 75 g a.i. /ha, 0.75 + 0.75 g a.i. /ha, 0.25 + 0.25 g a.i. /ha, 0.15 + 0.15 g a.i. /ha, 0.05 + 0.05 g a.i. /ha, 0.025 + 0.025 g a.i. /ha and 0.0025 + 0.0025 g a.i. /ha.

For the ternary mixture phosphorous acid equivalent + mandipropamid + mefenoxam, the dose range studied was: 1,500 + 75 + 75 g a.i. /ha, 150 + 0.75 + 0.75 g a.i. /ha, 50 + 0.25 + 0.25 g a.i. /ha, 30 + 0.15 + 0.15 g a.i. /ha, 10 + 0.05 + 0.05 g a.i. /ha, 5 + 0.025 + 0.025 g a.i. /ha and 0.5 +0.0025 + 0.0025 g a.i. /ha.

The UTC plants were only treated with water without any active compound.

For managing vapour effect of mefenoxam, all the different factors were separated and isolated after treatment all along the test in independent greenhouse boxes.

Four days before treatment, phosphorous acid was applied at 1,500 g a.i. /ha on plants planned to be treated later with phosphorous acid based product alone or in mixture.

A three days interval separated treatment and inoculation. Three days after the treatment, each plant was inoculated by spraying an aqueous suspension of sporangia of *Plasmopara viticola* obtained from infected leaves and sensitive to all the tested compounds. The concentration of sporangia was about 100,000 units per ml.

After contamination the plants were incubated for 7 days at 22°C in a saturated atmosphere. Seven days after contamination, symptoms were evaluated in terms of the extent of the lower surface of the leaves infected, in comparison with untreated (UTC) but contamined plants.

The efficacy of the treatment was calculated using the Abbott formula :

$$\text{efficacy} = (\text{ untreated - treated / untreated })\text{x } 100$$

The concentrations of the fungicide active substances alone or in mixture giving % efficacy for each component were determined based on the sigmoid curve dose / response model with their confidence intervals. The analysis of the results was made according to the TAMMES model (Isoboles, a graphic representation of synergism in pesticides, Neth.J. Plant Path. 70 (1964): 73-80).

The ED90, ED70 or ED50 values observed for each compound alone or in mixture were then inserted in a graphic representation as described by TAMMES.

Each of the components is expressed in a coordinate. The line which connects the points is called an isobole. With an isobole, the effect of different proportions of the components can be calculated. The observed value for the mixture corresponding to one ratio A / B is compared to the theoretical value calculated via the isobole of additive effect. In that case, the total effect observed is equal to the sum of the effects of the components taken independently.

A synergistic effect or a cooperative action of two or more compounds in mixture means that the total effect is greater or more prolonged than the sum of effects of the products taken independently.

Results:

The calculated results corresponding to ED90 or ED70 or ED50 (effective doses providing 90% or 70% or 50% of disease control) for the ratio have been calculated on the basis of 4 repeats per factor.

The results obtained are presented in the form of points values, corresponding to 90% or 70% or 50% control of the pathogen and placed in a TAMMES isobole diagram which comprises on the x-axis the doses of phosphorous acid equivalent expressed in g a.i. /ha and on the y-axis the doses of the mixture mandipropamid with mefenoxam also in g a.i. /ha.

For the ratio 20 / 1 / 1 corresponding to phosphorous acid with mandipropamid and mefenoxam, the preventive test done on grapevine downy mildew shows synergistic effect of the compositions containing compounds phosphorous acid with mandipropamid and mefenoxam. It has been demonstrated that less amount of both compounds are necessary to effectively control 90%, 70% and 50% of the disease compared to the theoretical doses of each compounds expected in the mixture with just additional effect.

In tables 2 and 3, EC50, EC70, EC90 calculated according to efficacy observed for this ratio illustrate these results.

Table 2 : Treatment of vine cuttings with a composition according to the invention - Practical 90%, 70% and 50% of disease control (ED50, ED70, ED90) observed for the mixture of phosphorous acid with mandipropamid and mefenoxam

| | ED50 g a.i. / ha | ED70 g a.i. / ha | ED90 g a.i. / ha |
|---|---|---|---|
| A : phosphorous acid | 500 | 650 | 1,500 |
| B + C : mandipropamid + mefenoxam | 0.034 | 0.053 | 0.11 |

Table 3 : Treatment of vine cuttings with a composition according to the invention - Synergism of a mixture of phosphorous acid with mandipropamid and mefenoxam according to TAMMES method for 90%, 70% and 50% of disease control.

| | phosphorous acid + mandipropamid + mefenoxam g a.i. / ha | mandipropamid + mefenoxam g a.i. / ha | phosphorous acid g a.i. / ha |
|---|---|---|---|
| Ratio | 20 / 1 / 1 | 1 / 1 | / |
| Practical dose for 90% efficacy | 0.9 / 0.045 / 0.045 (= 0.99) | 0.055 / 0.055 (= 0.11) | 1,500 |
| Theoretical dose for 90% efficacy | 1.1 / 0.055 / 0.055 (= 1.21) | / | / |
| Synergy for 90% efficacy (1-practical dose / theoretical dose) | 20% | | |
| Practical dose for 70% efficacy | 0.25 / 0.0125 / 0.0125 (= 0.275) | 0.026/0.026 (= 0.053) | 650 |
| Theoretical dose for 70% efficacy | 0.53 / 0.026 / 0.026 (= 0.582) | / | / |
| Synergy for 70% efficacy (1-practical dose / theoretical dose) | 50% | | |
| Practical dose for 50% efficacy | 0.11 / 0.0055 / 0.0055 (= 0.121) | 0.017 / 0.017 (= 0.034) | 500 |
| Theoretical dose for 50% efficacy | 0.34 / 0.017 / 0.017 (= 0.374) | / | / |
| Synergy for 50% efficacy (1-practical dose / theoretical dose) | 70% | | |

Conclusion

In these greenhouse studies, synergy for a mixture of phosphorous acid wit mandipropamid and mefenoxam was demonstrated in 72h preventive situation for the ratio 20 / 1 / 1 according to TAMMES model.

**Claims**

**1.** A fungicide composition comprising:

A) a phosphorous acid derivative selected from fosetyl-Al and phosphorous acid;

B) mandipropamid; and
C) a further fungicide compound selected from folpet and mefenoxam

wherein the weight ratios of the compounds A : B : C are from the range 1 : 0.01 : 0.01 to the range 1 : 0.1 : 2.

**2.** A composition according to claim 1 comprising fosetyl-Al, mandipropamid and folpet or mefenoxam.

**3.** A composition according to claims 1 to 2 wherein the weight ratios of the compounds A : B : C are from the range 1 : 0.01 : 0.01 to the range 1 : 0.1 : 0.01.

**4.** A method for curatively or preventively controlling the phytopathogenic fungi of plants or crops comprising the use of a fungicide composition according to claims 1 to 3 by application to the seed, the plant or to the fruit of the plant or to the soil in which the plant is growing or in which it is desired to grow.

**5.** A method of treatment for curatively or preventively controlling the phytopathogenic fungi of plants or crops comprising the use of

A) fosetyl-Al used in an amount from 500 to 2,000 g/ha;
B) mandipropamid used in an amount from 20 to 150 g/ha; and
C) a further fungicide compound selected from folpet used in an amount from 500 to 1,000 g/ha, and mefenoxam used in an amount from 50 to 120 g/ha,

wherein the weight ratios of the compounds A : B : C are from the range 1 : 0.01 : 0.01 to the range 1 : 0.1 : 2.

**6.** A method according to claim 5 comprising the use of

A) fosetyl-Al used in an amount from 500 to 2,000 g/ha;
B) mandipropamid used in an amount from 50 to 75 g/ha; and
C) a further fungicide compound selected from folpet used in an amount from 500 to 750 g/ha and mefenoxam used in an amount from 50 to 75 g/ha,

wherein the weight ratios of the compounds A : B : C are from the range 1 : 0.01 : 0.01 to the range 1 : 0.1 : 2.

## Patentansprüche

**1.** Fungizidzusammensetzung, die Folgendes umfasst:

A) ein Derivat der phosphorigen Säure, ausgewählt aus Fosetyl-Al und phosphoriger Säure;
B) Mandipropamid; und
C) eine weitere Fungizidverbindung, ausgewählt aus Folpet und Mefenoxam,

wobei die Gewichtsverhältnisse der Verbindungen A:B:C im Bereich von 1:0,01:0,01 bis im Bereich von 1:0,1:2 liegen.

**2.** Zusammensetzung nach Anspruch 1, die Fosetyl-Al, Mandipropamid und Folpet oder Mefenoxam umfasst.

**3.** Zusammensetzung nach den Ansprüchen 1 bis 2, wobei die Gewichtsverhältnisse der Verbindungen A:B:C im Bereich von 1:0,01:0,01 bis im Bereich von 1:0,1:0,01 liegen.

**4.** Verfahren zur kurativen oder präventiven Bekämpfung von phytopathogenen Pilzen von Pflanzen oder Kulturen, bei dem man eine Fungizidzusammensetzung nach den Ansprüchen 1 bis 3 durch Ausbringung auf den Samen, auf die Pflanze oder auf die Frucht der Pflanze oder auf den Boden, in dem die Pflanze wächst oder wachsen soll, verwendet.

**5.** Verfahren zur kurativen oder präventiven Bekämpfung von phytopathogenen Pilzen an Pflanzen oder Kulturen, bei dem man

A) Fosetyl-Al in einer Aufwandmenge von 500 bis 2 000 g/ha;
B) Mandipropamid in einer Aufwandmenge von 20 bis 150 g/ha; und
C) eine weitere Fungizidverbindung, ausgewählt aus Folpet in einer Aufwandmenge von 500 bis 1 000 g/ha und Mefenoxam in einer Aufwandmenge von 50 bis 120 g/ha,

verwendet, wobei die Gewichtsverhältnisse der Verbindungen A:B:C im Bereich von 1:0,01:0,01 bis im Bereich von 1:0,1:2 liegen.

**6.** Verfahren nach Anspruch 5, bei dem man

A) Fosetyl-Al in einer Aufwandmenge von 500 bis 2 000 g/ha;
B) Mandipropamid in einer Aufwandmenge von 50 bis 75 g/ha; und
C) eine weitere Fungizidverbindung, ausgewählt aus Folpet in einer Aufwandmenge von 500 bis 750 g/ha und Mefenoxam in einer Aufwandmenge von 50 bis 75 g/ha,

verwendet, wobei die Gewichtsverhältnisse der Verbindungen A:B:C im Bereich von 1:0,01:0,01 bis im Bereich von 1:0,1:2 liegen.

## Revendications

**1.** Composition fongicide comprenant :

A) un dérivé d'acide phosphoreux choisi parmi fosétyl-Al et l'acide phosphoreux ;
B) mandipropamide ; et
C) un composé fongicide supplémentaire choisi parmi le folpet et le méfénoxam

dans laquelle les rapports en poids des composés A : B : C sont de la plage de 1:0,01:0,01 à la plage de 1:0,1:2.

**2.** Composition selon la revendication 1 comprenant le fosétyl-Al, le mandipropamide et le folpet ou le méfénoxam.

**3.** Composition selon les revendications 1 à 2 dans laquelle les rapports en poids des composés A : B : C sont dans la plage de 1:0,01:0,01 à la plage de 1:0,1:0,01.

**4.** Procédé de lutte curative ou préventive contre les champignons phytopathogènes de plantes ou de cultures comprenant l'utilisation d'une composition fongicide selon les revendications 1 à 3 par application sur la graine, la plante ou le fruit de la plante ou sur le sol dans lequel la plante est cultivée ou dans lequel on souhaite la cultiver.

**5.** Procédé de traitement pour lutter de façon curative ou préventive contre les champignons phytopathogènes de plantes ou de cultures comprenant l'utilisation de

A) fosétyl-Al utilisé en une quantité de 500 à 2 000 g/ha ;
B) mandipropamide utilisé en une quantité de 20 à 150 g/ha ; et
C) un autre composé fongicide choisi parmi le folpet utilisé en une quantité de 500 à 1 000 g/ha, et le méfénoxam utilisé en une quantité de 50 à 120 g/ha,

dans lequel les rapports en poids des composés A : B : C sont de la plage de 1:0,01:0,01 à la plage de 1:0,1:2.

**6.** Procédé selon la revendication 5 comprenant l'utilisation de

A) fosétyl-Al utilisé en une quantité de 500 à 2 000 g/ha ;
B) mandipropamide utilisé en une quantité de 50 à 75 g/ha ; et
C) un autre composé fongicide choisi parmi le folpet utilisé en une quantité de 500 à 750 g/ha et le méfénoxam utilisé en une quantité de 50 à 75 g/ha,

dans lequel les rapports en poids des composés A : B : C sont dans la plage de 1:0,01:0,01 à la plage de 1:0,1:2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0187822 A1
- WO 03041728 A
- WO 20040459804 A2
- WO 2005041653 A2
- EP 230209 A2
- WO 2004047540 A2


### Non-patent literature cited in the description


- **COUCH ; SMITH.** *Crop Prot.,* 1991, vol. 10 (5), 386-390
- Isoboles, a graphic representation of synergism in pesticides. *Neth.J. Plant Path.,* 1964, vol. 70, 73-80